# EUROPEAN PATENT SPECIFICATION

(11) **EP 4 070 827 B1**
(45) Date of publication and mention of the grant of the patent: **20.05.2026**
(21) Application number: 22164600.3
(22) Date of filing: 28.03.2022
(51) Int. Cl.: A61M 3/02

(54) **IN-FLOW CONTROL FOR PASSIVELY IRRIGATED ELECTROPHYSIOLOGY DEVICES**
EINSTRÖMUNGSREGELUNG FÜR PASSIV BEWÄSSERTE ELEKTROPHYSIOLOGISCHE VORRICHTUNGEN
COMMANDE DE DÉBIT ENTRANT POUR DISPOSITIFS D'ÉLECTROPHYSIOLOGIE IRRIGUÉS PASSIVEMENT

(30) Priority: 29.03.2021 US 202117215509
(43) Date of publication of application: 12.10.2022
(73) Proprietor: Biosense Webster (Israel) Ltd, Yokneam, 2066717 (IL)
(72) Inventor: TANG, Raymond Yue-Sing, Irvine, 92618 (US)
(74) Representative: Carpmaels & Ransford LLP

(56) References cited:
- EP-B1- 3 131 617
- US-A1- 2019 138 032

## Description

### FIELD OF INVENTION

This invention relates to irrigation systems and tubing for delivering fluid through a patient's vasculature, in particular when a patient is undergoing catheter-based cardiac ablation procedures in which irrigation fluid is delivered to the tissue ablation site in the heart through tubing that extends through a catheter.

### BACKGROUND

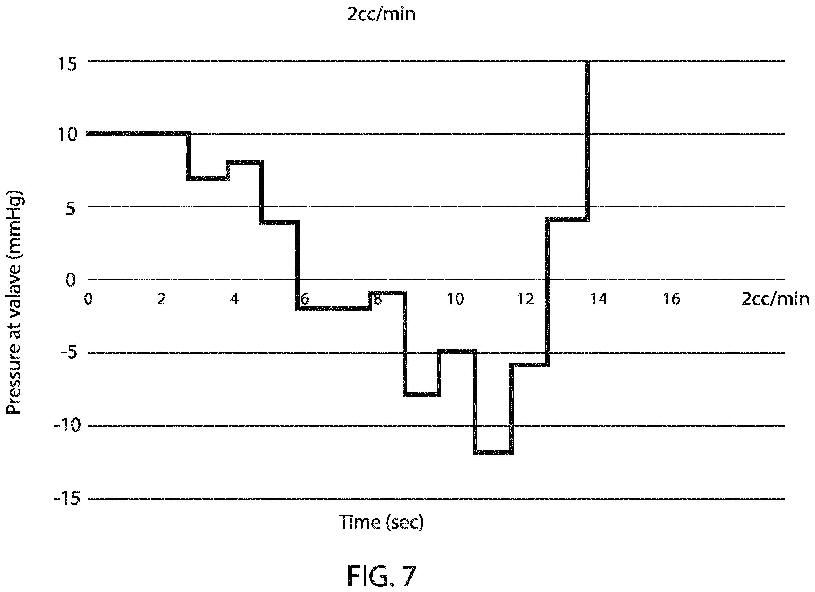

With irrigated EP devices, irrigation fluid, e.g., saline, is sourced from a fluid bag that is punctured by a bag spike of a drip chamber that is connected to an irrigation tubing whose distal end is typically connected to a luer hub or side port provided at or near a proximal end of a guiding sheath. For a passively-irrigated sheath where irrigation is supplied without a pump, it is desirable for the irrigation pathway to from a saline bag to the sheath to maintain positive pressure so as to ensure fluid flow for minimizing risks of blood coagulation. With the sheath inserted in a patient's vasculature and irrigated by the saline bag, the pressure in the irrigation pathway as measured, e.g., at a side port of sheath, would depend on a height differential of the saline bag relative to the height of the patient minus the cardia pressure. The pressure in the irrigation pathway is normalized but varies when a catheter is inserted into or removed from the guiding sheath. In particular, when a catheter is inserted into the sheath, a vacuum, as measured at a location proximal of the sheath, is created in the irrigation pathway trailing the catheter as it is advanced through the sheath until the catheter tip passes the distal tip of the sheath, catheter is retracted from the sheath, a pressure rise occurs in the irrigation pathway as measured from the location proximal of the sheath. This variance is depicted in **FIG. 7** which shows the change in pressure of irrigation flow at a rate of 2cc/min so measured during a catheter insertion and retraction. Where the irrigation flow is insufficient to compensate for the change of negative pressure, there is possibility for air entrainment within the sheath which poses a significant risk for the patient.

Accordingly, there is a desire for a device that is responsive to changes in irrigation flow pressure and self-adjusts to normalize the fluid flow rate in an irrigation pathway between a saline bag and a sheath or other electrophysiology device such as a catheter.
EP3131617B1 discloses systems and methods that generally involve shunting fluid, e.g., shunting cerebrospinal fluid in the treatment of hydrocephalus. Catheters with built-in flow indicators are provided. Exemplary flow indicators include projections that extend radially inward from the interior surface of the catheter and which include imageable portions (e.g., portions which are visible under magnetic resonance imaging (MRI)). Movement of the flow indicators caused by fluid flowing through the catheter can be detected using MRI, thereby providing a reliable indication as to whether the catheter is partially or completely blocked. Systems and methods for flushing a shunt system are also disclosed herein, as are various systems and methods for opening auxiliary fluid pathways through a shunt system.

### SUMMARY OF THE INVENTION

The invention is defined in the independent claims, embodiments of the invention are defined in the dependent claims.

### BRIEF DESCRIPTION OF THE DRAWINGS

These and other features and advantages of the present invention will be better understood by reference to the following detailed description when considered in conjunction with the accompanying drawings. It is understood that selected structures and features have not been shown in certain drawings so as to provide better viewing of the remaining structures and features.
FIG. 1 is an illustration depicting an in-line flow control device in use with irrigation tubing for delivering fluid to an electrophysiology apparatus, for example, a guiding sheath.
FIG. 2A is perspective view of an in-line flow control device, according to an embodiment, with first and second members enclosing a diaphragm (not shown).
FIG. 2B is a perspective view of the in-line flow control device of FIG. 2A, with the second member and the diaphragm but without the first member.
FIG. 2C is a perspective view of the in-line flow control device of FIG. 2A, with the second member but without the first member and the diaphragm.
FIG. 3 is a side cross-sectional view of the in-line flow control device of FIG. 2A, in a neutral configuration allowing a fluid flow.
FIG. 3A is a detailed view of a portion of in-line flow control device of FIG. 3.
FIG. 4 is a side cross-sectional view of the in-line flow control device of FIG. 2A in a first flexed configuration allowing a greater fluid flow.
FIG. 5 is a side cross-sectional view of the in-line flow control device of FIG. 2A in a second flexed configuration stopping fluid flow.
FIG. 6A is a side cross-sectional view of an in-line flow control device, according to another embodiment, in a neutral configuration.
FIG. 6B is the in-line flow control device of FIG. 6A, in a first flexed configuration.
FIG. 6C. is the in-line flow control device of FIG. 6A in a second flexed configuration.
FIG. 7 is a graph of changes in pressure of an irrigation pathway as measured from a side port of a guiding sheath during catheter insertion and retraction therethrough.

### DETAILED DESCRIPTION OF THE INVENTION

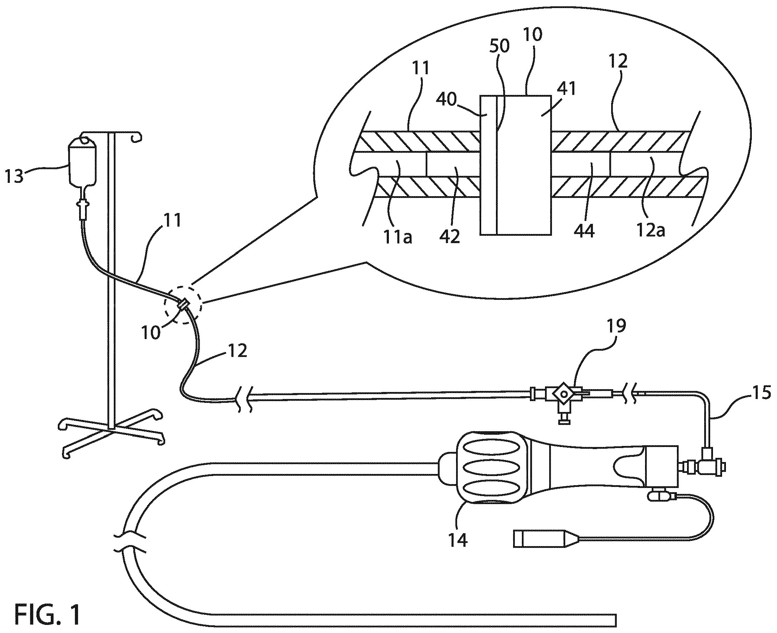

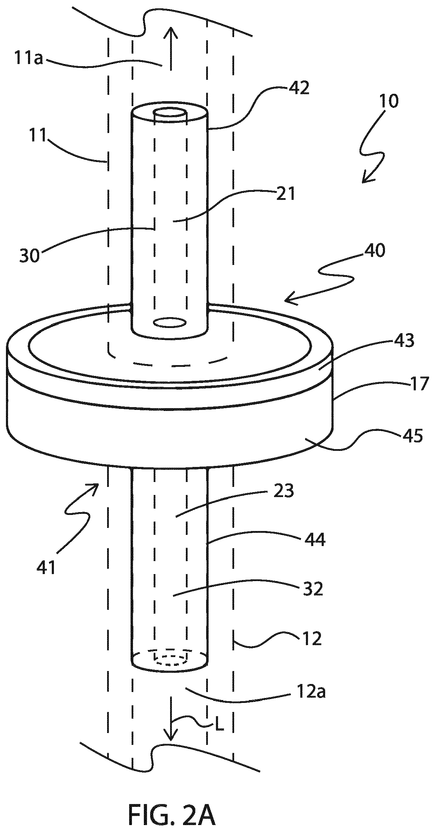

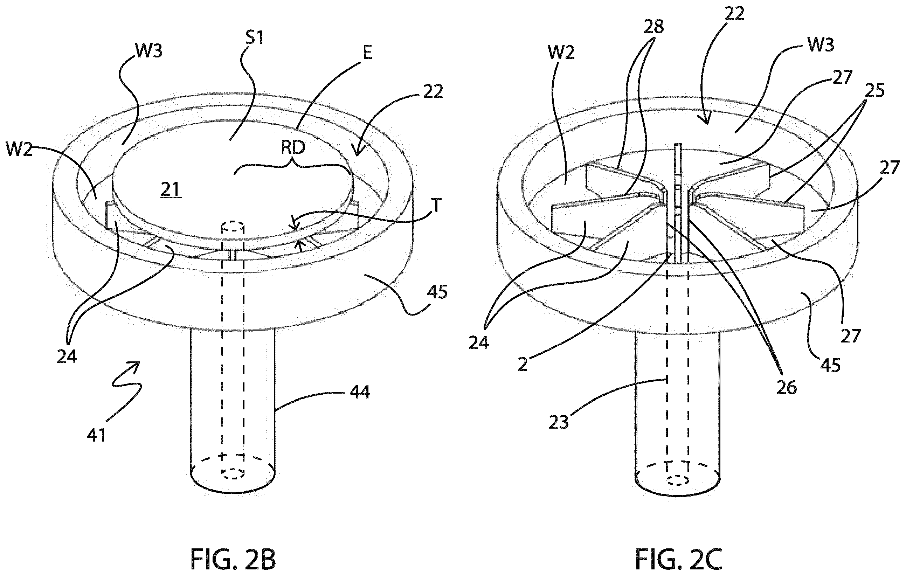

In some embodiments, as shown in **FIG. 1****,** an in-line flow control device 10 is configured for use and connection between a proximal tubing 11 and a distal tubing 12, each of which has a respective lumen 11a, 12a through which fluid is passed. For example, the proximal tubing 11 may receive irrigation fluid, such as saline, from a fluid bag 13, and the distal tubing 12 may pass the irrigation fluid to an irrigated electrophysiology (EP) device 14, e.g., a guiding sheath, which has an irrigation tubing 15 through which irrigation fluid is passed and a catheter may be inserted and retracted. With reference to **FIG. 2A****,** **FIG. 2B and FIG. 2C****,** the device 10 has a housing 17 with a center longitudinal axis L that defines a fluid path that includes an elastically flexible diaphragm 20, an upstream path 30 before the diaphragm 20 and a downstream path 32 after the diaphragm 20, where the diaphragm is responsive to changes in liquid pressure and air pressure upstream and downstream of the diaphragm in self-adjusting the amount of flow and/or limiting the direction of flow through the device. As best seen in **FIG. 2B****,** the diaphragm 20 is generally circular defined by circumferential outer edge E, radius RD, thickness T, first or ingress surface S1 and second or egress surface S2.

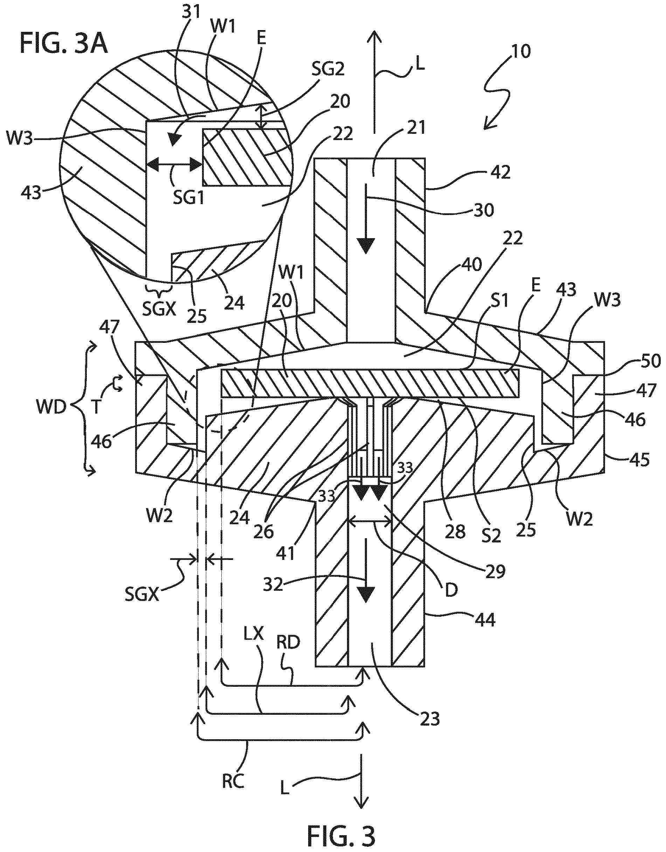

With further reference to **FIG. 3****,** the device 10 includes a proximal lumen 21 that defines the upstream path 30, a diaphragm chamber 22 that houses the diaphragm 20 and a distal lumen 23 that defines the downstream path 32, where the proximal lumen 21 is configured to receive the fluid from the proximal tubing 11 **(****FIG. 1****)** and the distal lumen 23 is configured to pass the fluid to the distal tubing 12 **(****FIG. 1****).** The diaphragm chamber 22 has a first or ingress sidewall W1, a second or egress sidewall W2, a radial wall W3 therebetween, all together which surround a volume defined by a generally circular cross of radius RC transverse to the longitudinal axis L and a maximum width WD along the longitudinal axis L, where both the radius RC is greater than the diaphragm radius RD, and the width WD is greater than the diaphragm thickness T so as to accommodate the diaphragm 20 within the chamber 22 and allow fluid flow around and past the diaphragm 20 as part of the fluid path within the device 10.

The diaphragm 20 is supported in the chamber 22 such that its first surface S1 faces the proximal lumen 21 so the fluid (liquid or air) of the upstream path 30 can encounter the first surface S1 and act on the diaphragm 20, and its second surface S2 faces the distal lumen 23 so that any backflow of fluid (liquid or air) in the opposite direction downstream of the diaphragm 20 in the distal lumen 23 can encounter the second surface S2 and act on the diaphragm 20. Notably, the diaphragm is not affixed to any surface or feature inside the chamber (or at least its peripheral edge remains unaffixed and unattached inside the chamber) but rather rests under its own weight with its second surface S2 supported on raised projections or fins 24 that extend upwardly from the second sidewall W2 into the diaphragm chamber 22.

In some embodiments, each fin 24 lies on a radial line of the second sidewall W2, evenly spaced from its adjacent fins about the longitudinal axis L. In some embodiments, there are eight fins, each spaced at 45 degrees from the adjacent fin about the longitudinal axis L. Each fin 24 has an outer edge 25 and an inner edge 26. The outer edge 25 is configured and sized to provide a gap space SGX between it and radial wall W3. The inner edge 26 is separated from an inner edge 26 of a diametrically opposing fin 24 by a gap space about equal to a diameter D of the distal lumen 23 of the downstream path 32. Each fin 24 has a length LX that is less than the chamber radius RC, and a tapered profile such that it has a taller axial dimension at its inner edge 26 and a shorter axial dimension at its outer edge 25. Between the inner edges 26 of the fins is a proximal opening 29 of the distal lumen 23.

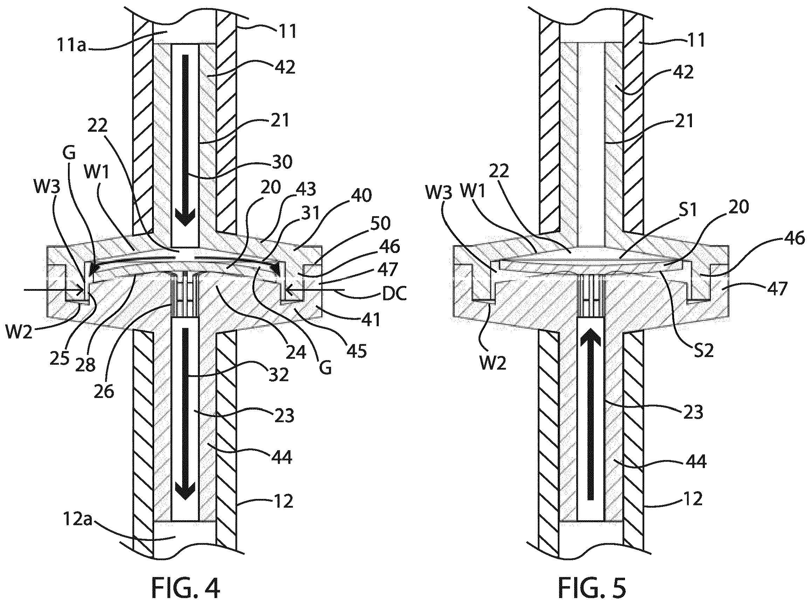

The diaphragm 20 in its neutral configuration is generally planar and therefore rests under its own weight on the fins 24 at or near the inner edges 26, with the outer edge E and adjacent portion thereof to extend free from contact with either the fins 24 and the first sidewall W1 of the chamber 22, best shown in **FIG. 3A****,** leaving a first predetermined (e.g., generally horizontal) space gap SG1 between the outer edge E and the first sidewall W1 and/or a second predetermined (e.g., generally vertical) space gap SG2 between the first surface S1 and the first sidewall W1. This configuration of the diaphragm 20 allows the fluid entering the diaphragm chamber 22 from the proximal lumen 21 (along the upstream path 30) to encounter and flow over the first surface S1, continue around the outer edge E past the diaphragm 20 (see arrow 31 in **FIG.** 3A) and enter the flow channels 27 between the fins 24 (see arrows 33 in **FIG.** 3). With the diaphragm 20 in this neutral configuration, the device 10 allows fluid flow at low pressure due to the smaller space gap SG relative to the space gap provided with the diaphragm 20 is in a first flexed configuration of **FIG. 4****,** as described further below.

Where the fluid entering the proximal lumen 21 surpasses a predetermined threshold flow (e.g., a threshold flow rate), the fluid distorts and depresses the diaphragm 20 toward or into a first flexed configuration with the outer edge E and adjacent portion thereof flexed toward the fins 24, as shown in **FIG. 4****,** which increases the space gap SG1 and/or the space gap SG2 which allows a greater volume of fluid (see arrow 31 in **FIG. 4****)** to enter flow channels 27, thereby increasing the flow rate through the device 10. Likewise, negative pressure downstream of the diaphragm 20 in the distal lumen 23 may create a vacuum that draws the diaphragm 20 towards or onto the fins 24 which also increases the space gap SG1 and/or the space gap SG2. Negative pressure, e.g., when measured from a side port 19 **(****FIG. 1****),** can occur when the catheter 14 is inserted into, e.g., a guiding sheath, as understood by one of ordinary skill in the art.

Where the fluid entering the proximal lumen 21 subsequently decreases and falls below the predetermined threshold flow, the diaphragm 20 being subjected to lesser fluid pressure from the proximal lumen 21 elastically rebounds and returns toward its neutral (unflexed) configuration with the space gaps returning to their original dimensions, as shown in **FIG. 3****.**

Where fluid enters the chamber 22 from the distal lumen 23, such as from backflow of irrigation fluid or when the catheter is retracted partially or fully from the sheath and creates a trailing vacuum in the distal lumen 23 that surpasses the predetermined threshold flow, the diaphragm 20 distorts and is depressed into a second flexed configuration, as shown in **FIG. 5** (opposite of the first flexed configuration of **FIG. 4****)** with the outer edge E flexed away from the fins 24 and toward the first sidewall W1 whereupon contact with the first sidewall W1 the space gap SG2 therebetween is reduced to zero. As such, backflow fluid (liquid or air) is blocked from entering the proximal lumen 21. Likewise, positive pressure downstream of the diaphragm 20 in the distal lumen 23 may create a force that pushes the diaphragm away from the fins 24 which also decreases at least the space gap SG2. Positive pressure, e.g., when measured from the side port 19 (see **FIG. 1****),** can occur when the catheter is retracted from the guiding sheath.

Where the backflow fluid subsequently decreases and falls below the predetermined threshold flow, the diaphragm 20 being subjected to lesser fluid pressure from the distal lumen 23 elastically rebounds and returns toward its neutral (unflexed) configuration with the space gap SG2 returning to its original dimension, as shown in **FIG. 3****.**

With reference to **FIG. 3****,** in some embodiments, the housing of the device 10 has a construction that includes first and second members 40 and 41 which engage each other to form a fluid-tight seal 50 circumferentially around the diaphragm chamber 22. The first member 40 has a proximal tubular portion 42 that surrounds the proximal lumen 21, and a first concave portion 43 that surrounds generally a proximal half of the diaphragm chamber 22. The second member 41 has a distal tubular portion 44 that surrounds the distal lumen 23, and a second concave portion 45 that surrounds generally a distal half of the diaphragm chamber 22r. A first circumferential flange 46 of the first concave portion 43 overlaps with a second circumferential flange 47 of the second concave portion 45 to form the fluid-tight seal 50. In some embodiments, the concavity of the first sidewall W1 of the first member 40 provides an ideal surface with which the outer edge E of the diaphragm can contact and form a seal against backflow when the diaphragm 20 is in the second flexed configuration, as shown in **FIG. 5****.**

In use, the device 10 may be assembled by laying the diaphragm 20 on the raised projections or fins 24 of the second member 41 of the housing 17 with the diaphragm 20 centered relative to the distal lumen 23, as shown in **FIG. 2B****).** The first member 40 can then be placed over the second member 41 enclosing the diaphragm 20 within the chamber 22 and fitting the flange 46 of the first member 40 to sit within the flange 47 of the second member 41 in an interference fit to seal the diaphragm chamber 22. So assembled, as shown in **FIG. 1****,** the device 10 is inserted between the proximal tubing 11 and the distal tubing 12 with the proximal tubular portion 42 of the first member 40 of the housing 17 inserted into the proximal tubing 11 and the distal tubular portion 44 of the second member 41 is inserted into the distal tubing 12, thereby providing fluid communication between the lumen 11a and the proximal lumen 21 and between the lumen 12a and the distal lumen 23. When the irrigated EP device 14 (e.g., a probe, sheath, catheter, etc.) with which the device 10 is in use is initially inserted into a patient's vascular, sufficient negative pressure in the distal lumen 23, as measured in a side port 19, causes the diaphragm 20 to flex toward the fins 24 which increase and widen the gap space SG1 and/or the gap space SG2 to allow more fluid to flow into the chamber 22, past the diaphragm 20 and out into the distal lumen 23, as shown in **FIG. 4****.**

When the pressure between the distal lumen 23 and the proximal lumen 21 generally equalizes, the diaphragm 20 returns to its neutral configuration with at least the space gap SG2 decreasing to allow a medium flow through the chamber 22, as shown in **FIG. 3****.**

With the diaphragm in the neutral configuration **(****FIG. 3****)** or in a first flex configuration with flexure toward the fins 24 **(****FIG. 4****),** fluid in the proximal tubing 11 passes into the proximal lumen 21 and enters the diaphragm chamber 22 where it encounters the first surface S1 of the diaphragm 22. The fluid flows radially outwards, towards the circumferential outer edge E of the diaphragm, and passes through the space gaps SG1 and SG2, and into the channels 27 between the fins 24. The fluid exits the chamber 22 through the distal lumen 23.

When the EP device 14 is retracted from the patient's vasculature, sufficient positive pressure in the distal lumen 23, causes the diaphragm 20 to flex away from the fins 24 **(****FIG. 5****)** which closes the space gap SG2 to protect against backflow of fluids from the distal lumen 23 into the proximal lumen 21.

It is understood that the device 10 may be placed anywhere along the irrigation pathway between the fluid source and the side port 19 (see FIG. 1) because the pressure change resulting from insertion and retraction of a catheter within the guiding sheath is propagated through the irrigation pathway between at least the fluid bag 13 and the side port 19 (FIG. 1).

Advantageously, the device 10 responds to changes in pressure in the fluid path upstream or downstream of the diaphragm in self-adjusting the fluid flow through the device to normalize the pressure change. It is understood that different fluid dynamics within the device allow for both changes in pressure and fluid flow upstream or downstream to affect and effectuate movement of the diaphragm and/or for movement of the diaphragm to affect and effectuate changes in pressure and fluid flow upstream and downstream, as needed or appropriate.

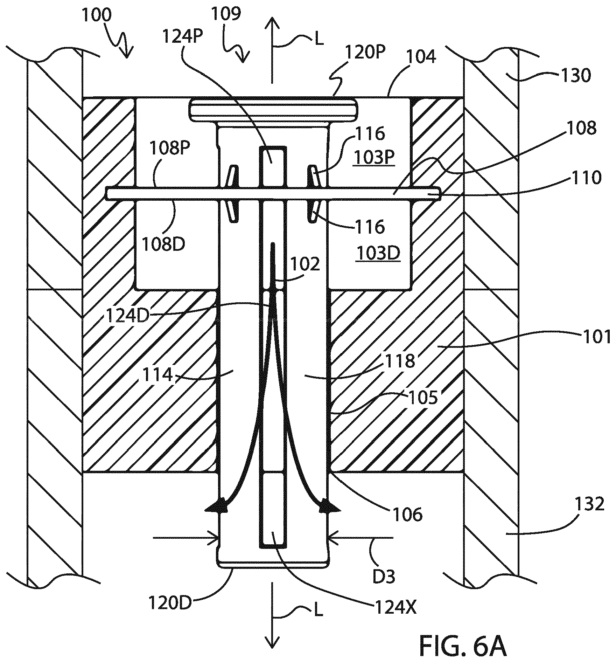

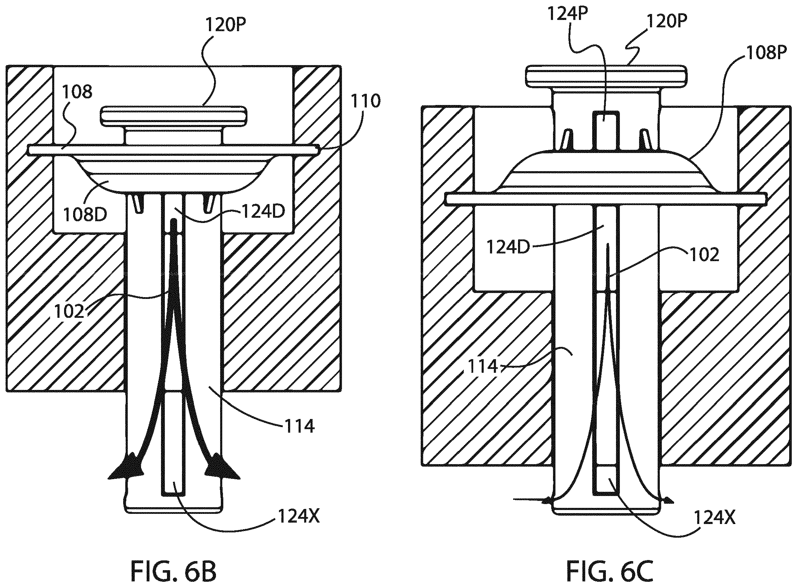

In alternate embodiments as shown in **FIG. 6A****,** **FIG. 6B** and **FIG. 6C****,** an in-line flow-control device 100 includes a housing 101 configured with a fluid pathway 102 that extends between an inlet 109 and an outlet 110 and defines a longitudinal axis L of the device. The fluid pathway flows through a chamber 103 and an elongated distal passage 105. The chamber 103 has a relatively larger diameter D1 and a proximal opening 104. The distal passage 105 has a relatively smaller diameter D2 and a distal opening 106. Inside the chamber 103 is a diaphragm 108 with a proximal surface 108P, a distal surface 108D, and a peripheral edge 110 that is affixed to an interior wall 112 of the chamber 103 which leaves a larger center portion of the diaphragm free to move and flex. Extending through a center opening of the diaphragm 108 is a piston 114 configured with a proximal portion 114P and a distal portion 114D, where the diaphragm 108 is fixed to the proximal portion by, for example, opposing fasteners or tabs 116 that extend from an outer surface of the piston immediately distal and proximal to the diaphragm to secure the diaphragm therebetween. The diaphragm divides and seals the chamber into portions, which may be two generally equal halves, namely, a proximal subchamber 103A and a distal subchamber 103B where the distal subchamber is 103B is in direct communication with the distal passage 105

The piston 114 is hollow with an interior enclosed by a sidewall 118, a proximal end wall 120P and a distal end wall. 120D. In some embodiments, the sidewall 118 is cylindrical and the end walls 120P, 120D are circular. The sidewall is configured with one or more elongated openings or slots 124 that extend longitudinally between the end walls 120P, 120D and allow fluid to enter into and exit from the interior of the piston. Each elongated slot 124 can be divided into a proximal opening 124P that is proximal of the diaphragm 108 and a distal opening 124D that is distal of the diaphragm 108. In some embodiments, the proximal opening 124P is relatively smaller/shorter and the distal opening 124D is relatively larger/longer. The sidewall 118 has an outer diameter D3 that is no greater and generally equal to the diameter D2 of the distal passage 105 so that the piston can slide freely in the distal passage 105 relative to the housing 101, along the longitudinal axis L, with generally a fluid-tight seal between the outer surface of the piston 114 and the side wall 118 of the distal passage 105.

The diaphragm 108 is constructed of an elastically flexible material so that it can assume different configurations, including: a neutral or planar configuration when the diaphragm is subjected to generally equal fluid pressures (liquid or air) on its first or proximal surface and its second or distal surface **(****FIG. 6A****),** and a first flexed or convex configuration **(****FIG. 6B****)** and a second or concave configuration **(****FIG. 6C****)** when it is subjected to unequal pressures on its first and second surfaces. As illustrated in these three figures, for each of the aforementioned configurations of the diaphragm, there is a corresponding position of the piston 114 relative to the housing 101. The piston 114 and each elongated slot opening 124 are configured such that a least a distal segment 124X of each distal opening 124D remains distal and exposed outside of the housing 101 in all the different configurations assumed by the diaphragm 108, but the size of the one or more exposed distal segments 124X can vary and differs with each diaphragm configuration. For example, when the diaphragm is in the neutral configuration **(****FIG. 6A****),** the exposed segment 124X is of a predetermined amount, when the diaphragm is in the convex configuration **(****FIG. 6B****),** the exposed segment 124X is of a relatively greater amount, and when the diaphragm is in the concave configuration **(****FIG.** 6C), the exposed segment 124X is of a relatively lesser amount.

Fluid can pass between the subchambers 103A, 103B only via the slot openings 124 and the interior of the piston 114. Fluid can pass between the proximal irrigation tubing 130 and the distal irrigating tubing 132 via the fluid pathway defined by the slot openings 124, the interior of the piston 114 and the exposed segment 124X.

In use, the device 100 is situated between a proximal irrigation tubing 130 and a distal irrigation tubing 132. In some embodiments, the device 100 is configured with an outer diameter that allows the device to fit within the lumens of the tubing 130 and 132. As fluid passes from the proximal irrigation tubing 130 into the inlet 109 and the chamber 103, the fluid enters the proximal subchamber 103A and enters the interior of the piston 114 via each proximal slot opening 124P upstream of the diaphragm 108. The fluid then passes through the interior of the piston 114 moving downstream of the diaphragm 108, and exits the piston114 and the housing 101 and into the distal irrigation tubing 132 solely through the exposed segment 124E of the distal portion 124D of each slot opening 124. The diaphragm 108 remains in the neutral configuration of **FIG. 6A** with the predetermined amount of exposed segment 124E of each slot opening 124D, so long as the pressure acting on the proximal surface 108P and the pressure acting on the distal surface 108D of the diaphragm remain generally equal and below the threshold force necessary to flex the diaphragm 108.

Where there is less pressure acting on the distal surface 108D of the diaphragm 108 relative to the pressure acting on the proximal surface 180P of the diaphragm, for example, when a catheter is advanced distally within a guiding sheath and creates a vacuum downstream or distal of the diaphragm, the diaphragm flexes convexly which pushes the piston distally and increases the size of the exposed segment 124E allowing a greater flow of fluid to pass into the distal irrigation tubing 132 compared to when the diaphragm is in its neutral/unflexed configuration. When the pressures distal and proximal of the diaphragm equalizes, the diaphragm returns to its neutral configuration, drawing the piston which decreases the size of the exposed segment 124E back to the initial predetermined size.

Where there is less pressure acting on the proximal surface 180P of the diaphragm 108 relative to the pressure acting on the distal surface 180D of the diaphragm, for example, when a catheter is retracted proximally within a guiding sheath and creates a vacuum upstream or proximal of the diaphragm, the diaphragm flexes concavely which retracts the piston proximally and decreases the size of the exposed segment 124E allowing a lesser flow of fluid to pass into the distal irrigation tubing 132 compared to when the diaphragm is in its neutral/unflexed configuration. When the pressures distal and proximal of the diaphragm equalizes, the diaphragm returns to its neutral configuration, advancing the piston which increases the size of the exposed segment 124E back to its initial predetermined size.

Accordingly, the device 101 automatically self-adjusts fluid flow passing through in accordance with pressure differences upstream and downstream of the diaphragm 108 to achieve pressure equilibrium in the chamber 103. It is understood that different fluid dynamics within the device allow for fluid flow in the piston to affect and effectuate changes the diaphragm and/or for changes in the diaphragm to affect and effectuate movement of the piston, as needed or appropriate.

In some embodiment, the proximal end wall 120P has a size or diameter greater than the sidewall 118 of the piston 114 as a safety feature in minimizing the risk of the piston dislodging from the diaphragm 197 and exiting the device 101.

The preceding description has been presented with reference to presently preferred embodiments of the invention. However, the scope of the present invention is defined by the claims and includes both combinations and subcombinations of the various features described hereinabove, as well as variations and modifications thereof which would occur to person skilled in the art upon reading the foregoing description and which fall within the scope of the claims.

## Claims

1. An inline flow control device (10) for passive irrigation tubing to be used with electrophysiology devices, comprises:
a proximal lumen (21);
a distal lumen (23);
a diaphragm (20) having a first surface and a second surface, the diaphragm housed in a chamber (22) having first and second opposing sidewalls (W1, W2), the first sidewall (W1) having a first opening in communication with the proximal lumen (21) and the second sidewall (W2) having a second opening in communication with the distal lumen (23);
wherein the device (10) is **characterized in that**:
the second sidewall of the chamber has a plurality of fins (24) configured to support
the diaphragm on its second surface,
wherein the diaphragm (20) is elastically flexible and at least unfixed at its peripheral edge within the chamber (22) so as to assume configurations, including a neutral configuration that allows a predetermined fluid flow through the device (10), a first flexed configuration that allows greater fluid flow through the device (10) and a second flexed configuration that generally ceases fluid flow through the device (10).

2. The device of claim 1, wherein the device defines a fluid path that includes through the proximal lumen (21), over the first surface of the diaphragm (S1), around a circumferential outer edge (E) of the diaphragm (20), under the second surface of the diaphragm (S2) and through the distal lumen (23).

3. The device of claim 1,wherein the fins (24) have a tapered profile, optionally wherein each fin (24) has a greater inner edge (26) and a lesser outer edge (25) to provide the tapered profile.

4. The device of claim 1, wherein the diaphragm (20) in the neutral configuration is generally planar.

5. The device of claim 1, wherein the diaphragm (20) in the first flexed configuration is flexed toward the second sidewall (W2).

6. The device of claim 1, wherein the diaphragm (20) in the second flexed configuration is flexed toward the first sidewall (W1).

7. The device of claim 1, wherein the second sidewall (W2) includes raised projections (24), optionally wherein the raised projections (24) are configured to support a lesser portion of the diaphragm (20) in the neutral configuration and a greater portion of the diaphragm (20) in the first flexed configuration.

8. The device of claim 1, wherein the raised projections (24) are configured to support a lesser portion of the diaphragm (20) in the second flexed configuration and a greater portion of the diaphragm (20) in the first flexed configuration.

9. An inline flow control device (100) for passive irrigation tubing to be used with electrophysiology devices, comprises:
a housing (101) having an inlet (109) and an outlet (110) and a chamber (103) and a distal passage (105) between the inlet (109) and the outlet (110);
an elastically deformable diaphragm (108) whose peripheral edge is fixed in the chamber (103), the diaphragm (108) configured to assume a neutral configuration, a first flexed configuration and
and wherein the inline flow control device (100) is **characterized by**:
a second flexed configuration assumed by the diaphragm; and
a hollow piston (114) extending through the diaphragm (108) and into the distal passage (105), the piston (114) configured for movement with the diaphragm (108) with a neutral position when the diaphragm (108) is in the neutral configuration, an extended position relative to the housing (101) when the diaphragm (108) is in the first flexed configuration and a retracted position relative to the housing (101) when the diaphragm is in the second flexed configuration, the piston (114) having a slot (124) opening with a proximal portion configured to pass fluid from the chamber (103) into an interior of the piston (114) and a distal portion configured to pass the fluid from the interior to the outlet (110), the distal portion having a variable exposed segment (124X) outside of the housing (103) that is greater when the piston (114) is in the extended position and lesser when the piston (114) is in the retracted position.

10. The device of claim 9, wherein the slot (124) opening has a proximal portion proximal of the diaphragm (108) and a distal portion distal of the diaphragm (108).

11. The device of claim 9, wherein the diaphragm (108) has a peripheral edge that is fixed to an inner surface of the chamber (103).

12. The device of claim 9, wherein the neutral configuration of the diaphragm (108) includes a generally planar configuration.

13. The device of claim 9, the first flexed configuration of the diaphragm (108) includes a convex flexure.

14. The device of claim 9, wherein the second flexed configuration of the diaphragm (108) includes a concave flexure.

15. The device of claim 9, wherein the piston (114) includes (i) a first slot opening and a second slot opening, or (ii) a first member on its outer surface proximal of the diaphragm (108) and a second member on its outer surface distal of the diaphragm (108), the first and second members configured to secure the piston (114) to the diaphragm (108).

## Patentansprüche

1. Inline-Durchflussregelvorrichtung (10) für eine Verschlauchung für eine passive Spülung, die mit elektrophysiologischen Vorrichtungen zu verwenden ist, die umfasst:
ein proximales Lumen (21);
ein distales Lumen (23);
eine Membran (20), die eine erste Oberfläche und eine zweite Oberfläche aufweist, wobei die Membran in einer Kammer (22), die eine erste und eine zweite gegenüberliegende Seitenwand (W1, W2) aufweist, untergebracht ist, wobei die erste Seitenwand (W1) eine erste Öffnung in Verbindung mit dem proximalen Lumen (21) aufweist und die zweite Seitenwand (W2) eine zweite Öffnung in Verbindung mit dem distalen Lumen (23) aufweist;
wobei die Vorrichtung (10) **dadurch gekennzeichnet ist, dass:**
die zweite Seitenwand der Kammer eine Vielzahl von Rippen (24) aufweist, die konfiguriert ist, um die Membran auf ihrer zweiten Oberfläche zu stützen,
wobei die Membran (20) elastisch biegbar und mindestens an ihrem Umfangsrand innerhalb der Kammer (22) unbefestigt ist, um Konfigurationen anzunehmen, einschließlich einer neutralen Konfiguration, die einen zuvor bestimmten Fluiddurchfluss durch die Vorrichtung (10) hindurch ermöglicht, einer ersten gebogenen Konfiguration, die einen größeren Fluiddurchfluss durch die Vorrichtung (10) hindurch ermöglicht, und einer zweiten gebogenen Konfiguration, die den Fluiddurchfluss durch die Vorrichtung (10) hindurch im Allgemeinen unterbricht.

2. Vorrichtung nach Anspruch 1, wobei die Vorrichtung einen Fluidpfad definiert, der durch das proximale Lumen (21) hindurch, über der ersten Oberfläche der Membran (S1), um einen umlaufenden Außenrand (E) der Membran (20) herum, unter der zweiten Oberfläche der Membran (S2) und durch das distale Lumen (23) hindurch einschließt.

3. Vorrichtung nach Anspruch 1, wobei die Rippen (24) ein sich verjüngendes Profil aufweisen, wobei jede Rippe (24) optional einen größeren Innenrand (26) und einen kleineren Außenrand (25) aufweist, um das sich verjüngende Profil bereitzustellen.

4. Vorrichtung nach Anspruch 1, wobei die Membran (20) in der neutralen Konfiguration im Allgemeinen planar ist.

5. Vorrichtung nach Anspruch 1, wobei die Membran (20) in der ersten gebogenen Konfiguration zu der zweiten Seitenwand (W2) hin gebogen ist.

6. Vorrichtung nach Anspruch 1, wobei die Membran (20) in der zweiten gebogenen Konfiguration zu der ersten Seitenwand (W1) hin gebogen ist.

7. Vorrichtung nach Anspruch 1, wobei die zweite Seitenwand (W2) erhabene Vorsprünge (24) einschließt, wobei die erhabenen Vorsprünge (24) optional konfiguriert sind, um einen kleineren Abschnitt der Membran (20) in der neutralen Konfiguration und einen größeren Abschnitt der Membran (20) in der ersten gebogenen Konfiguration zu stützen.

8. Vorrichtung nach Anspruch 1, wobei die erhabenen Vorsprünge (24) konfiguriert sind, um einen kleineren Abschnitt der Membran (20) in der zweiten gebogenen Konfiguration und einen größeren Abschnitt der Membran (20) in der ersten gebogenen Konfiguration zu stützen.

9. Inline-Durchflussregelvorrichtung (100) für die Verschlauchung für die passive Spülung, die mit elektrophysiologischen Vorrichtungen zu verwenden ist, die umfasst:
ein Gehäuse (101), das einen Einlass (109) und einen Auslass (110) und eine Kammer (103) und einen distalen Durchgang (105) zwischen dem Einlass (109) und dem Auslass (110) aufweist;
eine elastisch verformbare Membran (108), deren Umfangsrand in der Kammer (103) befestigt ist, wobei die Membran (108) konfiguriert ist, um eine neutrale Konfiguration, eine erste gebogene Konfiguration anzunehmen, und
wobei die Inline-Durchflussregelvorrichtung (100)
**gekennzeichnet ist durch:**
eine zweite gebogene Konfiguration, die **durch** die Membran angenommen wird; und
einen hohlen Kolben (114), der sich **durch** die Membran (108) hindurch und in den distalen Durchgang (105) hinein erstreckt, wobei der Kolben (114) für eine Bewegung mit der Membran (108) mit einer neutralen Position, wenn sich die Membran (108) in der neutralen Konfiguration befindet, einer ausgefahrenen Position relativ zu dem Gehäuse (101), wenn sich die Membran (108) in der ersten gebogenen Konfiguration befindet, und einer eingezogenen Position relativ zu dem Gehäuse (101), wenn sich die Membran in der zweiten gebogenen Konfiguration befindet, konfiguriert ist, wobei der Kolben (114) eine Schlitzöffnung (124) mit einem proximalen Abschnitt, der konfiguriert ist, um Fluid aus der Kammer (103) in ein Inneres des Kolbens (114) hinein zu leiten, und einen distalen Abschnitt, der konfiguriert ist, um das Fluid aus dem Inneren zu dem Auslass (110) zu leiten, aufweist, wobei der distale Abschnitt ein variables freigelegtes Segment (124X) außerhalb des Gehäuses (103) aufweist, das größer ist, wenn sich der Kolben (114) in der ausgefahrenen Position befindet, und kleiner ist, wenn sich der Kolben (114) in der eingezogenen Position befindet.

10. Vorrichtung nach Anspruch 9, wobei die Schlitzöffnung (124) einen proximalen Abschnitt proximal von der Membran (108) und einen distalen Abschnitt distal von der Membran (108) aufweist.

11. Vorrichtung nach Anspruch 9, wobei die Membran (108) einen Umfangsrand aufweist, der an einer Innenoberfläche der Kammer (103) befestigt ist.

12. Vorrichtung nach Anspruch 9, wobei die neutrale Konfiguration der Membran (108) eine im Allgemeinen planare Konfiguration einschließt.

13. Vorrichtung nach Anspruch 9, wobei die erste gebogene Konfiguration der Membran (108) eine konvexe Biegung einschließt.

14. Vorrichtung nach Anspruch 9, wobei die zweite gebogene Konfiguration der Membran (108) eine konkave Biegung einschließt.

15. Vorrichtung nach Anspruch 9, wobei der Kolben (114) (i) eine erste Schlitzöffnung und eine zweite Schlitzöffnung oder (ii) ein erstes Element an seiner Außenoberfläche proximal von der Membran (108) und ein zweites Element an seiner Außenoberfläche distal von der Membran (108) einschließt, wobei das erste und das zweite Element konfiguriert sind, um den Kolben (114) an der Membran (108) anzubringen.

## Revendications

1. Dispositif de commande d'écoulement en ligne (10) destiné à une tubulure d'irrigation passive à utiliser avec des dispositifs d'électrophysiologie, qui comprend :
une lumière proximale (21) ;
une lumière distale (23) ;
un diaphragme (20) ayant une première surface et une seconde surface, le diaphragme étant logé dans une chambre (22) ayant des première et seconde parois latérales (W1, W2) opposées, la première paroi latérale (W1) ayant une première ouverture en communication avec la lumière proximale (21) et la seconde paroi latérale (W2) ayant une seconde ouverture en communication avec la lumière distale (23) ;
dans lequel le dispositif (10) est **caractérisé en ce que** :
la seconde paroi latérale de la chambre a une pluralité d'ailettes (24) conçues pour supporter
le diaphragme sur sa seconde surface,
dans lequel le diaphragme (20) est élastiquement flexible et au moins non fixé au niveau de son bord périphérique au sein de la chambre (22) de façon à adopter des configurations, comportant une configuration neutre qui permet un écoulement de fluide prédéterminé à travers le dispositif (10), une première configuration fléchie qui permet un plus grand écoulement de fluide à travers le dispositif (10) et une seconde configuration fléchie qui arrête généralement un écoulement de fluide à travers le dispositif (10).

2. Dispositif selon la revendication 1, dans lequel le dispositif définit un chemin de fluide qui comporte à travers la lumière proximale (21), par-dessus la première surface du diaphragme (S1), autour d'un bord externe circonférentiel (E) du diaphragme (20), sous la seconde surface du diaphragme (S2) et à travers la lumière distale (23).

3. Dispositif selon la revendication 1, dans lequel les ailettes (24) ont un profil effilé, facultativement dans lequel chaque ailette (24) a un bord interne (26) plus grand et un bord externe (25) plus petit pour fournir le profil effilé.

4. Dispositif selon la revendication 1, dans lequel le diaphragme (20) dans la configuration neutre est généralement plan.

5. Dispositif selon la revendication 1, dans lequel le diaphragme (20) dans la première configuration fléchie est fléchi en direction de la seconde paroi latérale (W2).

6. Dispositif selon la revendication 1, dans lequel le diaphragme (20) dans la seconde configuration fléchie est fléchi en direction de la première paroi latérale (W1).

7. Dispositif selon la revendication 1, dans lequel la seconde paroi latérale (W2) comporte des parties saillantes surélevées (24), facultativement dans lequel les parties saillantes surélevées (24) sont conçues pour supporter une partie plus petite du diaphragme (20) dans la configuration neutre et une partie plus grande du diaphragme (20) dans la première configuration fléchie.

8. Dispositif selon la revendication 1, dans lequel les parties saillantes surélevées (24) sont conçues pour supporter une partie plus petite du diaphragme (20) dans la seconde configuration fléchie et une partie plus grande du diaphragme (20) dans la première configuration fléchie.

9. Dispositif de commande d'écoulement en ligne (100) destiné à une tubulure d'irrigation passive à utiliser avec des dispositifs d'électrophysiologie, qui comprend :
un logement (101) ayant une entrée (109) et une sortie (110) et une chambre (103) et un passage distal (105) entre l'entrée (109) et la sortie (110) ;
un diaphragme (108) élastiquement déformable dont le bord périphérique est fixé dans la chambre (103), le diaphragme (108) étant conçu pour adopter une configuration neutre, une première configuration fléchie et
et dans lequel le dispositif de commande d'écoulement en ligne (100) est **caractérisé par :**
une seconde configuration fléchie adoptée par le diaphragme ; et
un piston (114) creux s'étendant à travers le diaphragme (108) et dans le passage distal (105), le piston (114) étant conçu pour un déplacement avec le diaphragme (108) avec une position neutre lorsque le diaphragme (108) est dans la configuration neutre, une position étendue par rapport au logement (101) lorsque le diaphragme (108) est dans la première configuration fléchie et une position rétractée par rapport au logement (101) lorsque le diaphragme est dans la seconde configuration fléchie, le piston (114) ayant une ouverture à fente (124) avec une partie proximale conçue pour faire passer un fluide de la chambre (103) vers un intérieur du piston (114) et une partie distale conçue pour faire passer le fluide de l'intérieur vers la sortie (110), la partie distale ayant un segment exposé variable (124X) à l'extérieur du logement (103) qui est plus grand lorsque le piston (114) est dans la position étendue et plus petit lorsque le piston (114) est dans la position rétractée.

10. Dispositif selon la revendication 9, dans lequel l'ouverture à fente (124) a une partie proximale qui est proximale par rapport au diaphragme (108) et une partie distale qui est distale par rapport au diaphragme (108).

11. Dispositif selon la revendication 9, dans lequel le diaphragme (108) a un bord périphérique qui est fixé à une surface interne de la chambre (103).

12. Dispositif selon la revendication 9, dans lequel la configuration neutre du diaphragme (108) comporte une configuration généralement plane.

13. Dispositif selon la revendication 9, la première configuration fléchie du diaphragme (108) comporte une flexion convexe.

14. Dispositif selon la revendication 9, dans lequel la seconde configuration fléchie du diaphragme (108) comporte une flexion concave.

15. Dispositif selon la revendication 9, dans lequel le piston (114) comporte (i) une première ouverture à fente et une seconde ouverture à fente, ou (ii) un premier élément sur sa surface externe, proximal par rapport au diaphragme (108) et un second élément sur sa surface externe, distal par rapport au diaphragme (108), les premier et second éléments étant conçus pour attacher le piston (114) au diaphragme (108).
